(19) Europäisches Patentamt
European Patent Office
Office européen des brevets

(11) Veröffentlichungsnummer: **0 379 003 B1**

# (12) EUROPÄISCHE PATENTSCHRIFT

(45) Veröffentlichungstag der Patentschrift: **25.01.95**

(51) Int. Cl.$^6$: **C07D 333/38**, A01N 43/10

(21) Anmeldenummer: **90100149.5**

(22) Anmeldetag: **04.01.90**

## (54) Thiophen-2-carbonsäurederivate, Verfahren zu ihrer Herstellung und ihre Verwendung zur Bekämpfung unerwünschten Pflanzenwuchses.

(30) Priorität: **16.01.89 DE 3901074**

(43) Veröffentlichungstag der Anmeldung:
**25.07.90 Patentblatt 90/30**

(45) Bekanntmachung des Hinweises auf die Patenterteilung:
**25.01.95 Patentblatt 95/04**

(84) Benannte Vertragsstaaten:
**CH DE ES FR GB IT LI NL**

(56) Entgegenhaltungen:
**EP-A- 0 207 355**
**DE-A- 3 629 584**
**GB-A- 2 195 634**
**US-A- 3 536 473**

(73) Patentinhaber: **BASF Aktiengesellschaft**
**Carl-Bosch-Strasse 38**
**D-67063 Ludwigshafen (DE)**

(72) Erfinder: **Kober, Reiner, Dr.**
**Im Schlittweg 20**
**D-6701 Fussgoenheim (DE)**
Erfinder: **Seele, Rainer, Dr.**
**Leiblstrasse 3**
**D-6701 Fussgoenheim (DE)**
Erfinder: **Neubauer, Hans-Juergen, Dr.**
**Mozartstrasse 6**
**D-4400 Muenster-Hiltrup (DE)**
Erfinder: **Saupe, Thomas, Dr.**
**Kressenwiesenweg 13**
**D-6902 Sandhausen (DE)**
Erfinder: **Wuerzer, Bruno, Dr.**
**Ruedigerstrasse 13**
**D-6701 Otterstadt (DE)**
Erfinder: **Meyer, Norbert, Dr.**
**Dossenheimer Weg 22**
**D-6802 Ladenburg (DE)**
Erfinder: **Westphalen, Karl-Otto, Dr.**
**Mausbergweg 58**
**D-6720 Speyer (DE)**

EP 0 379 003 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue herbizid wirksame Thiophen-2-carbonsäurederivate der allgemeinen Formel I

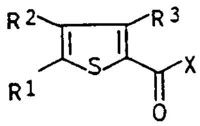

I,

in der die Substituenten folgende Bedeutung haben:

| | |
|---|---|
| $R^1$ bis $R^3$ | Wasserstoff, Halogen, verzweigtes oder unverzweigtes $C_1$-$C_6$-Alkyl, $C_1$-$C_8$-Alkoxy, $C_1$-$C_6$-Halogenalkyl, $C_1$-$C_6$-Halogenalkoxy, Nitro, Cyano, Phenyl oder ein- bis dreifach mit $C_1$-$C_4$-Alkyl, Halogen, $C_1$-$C_5$-Halogenalkyl, $C_1$-$C_4$-Alkoxy oder $C_1$-$C_5$-Halogenalkoxy-substituiertes Phenyl; |
| X | $-OR^4$ oder $-NH-OR^5$, |
| $R^4$ | verzweigtes oder unverzweigtes $C_3$-$C_{10}$-Alkinylalkyl, das unsubstituiert oder durch Halogen substituiert sein kann; |
| $R^5$ | verzweigtes oder unverzweigtes $C_1$-$C_5$-Alkyl, das unsubstituiert oder durch Halogen substituiert ist oder verzweigtes oder unverzweigtes $C_3$-$C_{10}$-Alkinylalkyl, das unsubstituiert oder durch Halogen substituiert sein kann; |

wobei Thiophen-2-carbonsäurepropargylester der Formel Ia

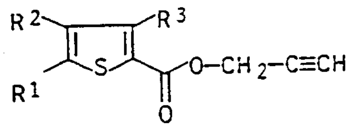

Ia,

ausgenommen sind, worin die Reste $R^1$ bis $R^3$ die folgende Bedeutung haben:

| $R^1$ | $R^2$ | $R^3$ |
|---|---|---|
| H | H | H |
| Cl | H | H |
| $O_2N-$ | H | H |
| Br | H | H |
| Br | Br | H |
| H | H | $H_3C-$ |
| $H_3C-$ | H | H |
| H | Br | H |
| -CN | H | H |
| $H_3C-$ | H | $H_3C-$ |
| $H_3CH_2C-$ | H | H |
| 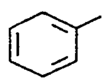 | H | H |
| 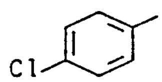 | H | H |
| $H_3CO-$ | H | H |
| H | H | $H_3CO-$ |
| H | H | Cl |
| Cl | H | Cl |
| Cl | Cl | H |
| Cl | Cl | Cl |

Außerdem betrifft die Erfindung ein verfahren zur Herstellung der Thiophen-2-carbonsäurederivate I sowie ihre Verwendung zur Bekämpfung unerwünschten Pflanzenwuchses.

Aus der Druckschrift Chim. Ther. 5, 32 (1970) sind Amide der 3,4,5-Trichlorthiophen-2-carbonsäure zur Bekämpfung von Parasiten bekannt.

In der US 3,536,473 werden halogenierte Thiophen-2-carbonsäuren und deren Salze sowie zur Säure hydrolysierbare Derivate wie Ester oder Amide als Mittel zur Regulierung des Pflanzenwachstums beschrieben. Die DE-A-36 29 584 lehrt ggf. substituierte Thiophen-2-carbonsäurepropargylesterals Schädlingsbekämpfungsmittel. Ein Hinweis auf andere z.B. herbizide Wirksamkeit ist dieser Schrift nicht zu entnehmen.

Der Erfindung lag die Aufgabe zugrunde, neue Thiophen-2-carbonsäurederivate mit guter herbizider Wirksamkeit zur Verfügung zu stellen. Weiterhin bestand die Aufgabe, neue biologische Wirkungen an sich bekannter Schädlingsbekämpfungsmittel aufzufinden.

Demgemäß wurden die eingangs definierten Thiophen-2-carbonsäurederivate der allgemeinen Formel I gefunden.

Die Substituenten in Formel I haben bevorzugt die folgende Bedeutung:

$R^1$ - $R^3$

Wasserstoff;

Halogen wie Fluor, Chlor, Brom oder Jod, insbesondere Fluor, Chlor oder Brom;

$C_1$-$C_6$-Alkyl, insbesondere $C_1$-$C_4$-Alkyl wie Methyl, Ethyl, ios-Propyl, n-Propyl, n-Butyl, iso-Butyl, sek.-Butyl, tert.-Butyl;

$C_1$-$C_8$-Alkoxy, insbesondere $C_1$-$C_4$-Alkoxy wie Methoxy, Ethoxy, Propyloxy, 1-Methylethoxy, Butoxy, 1-Methylpropyloxy, 2-Methylpropyloxy und 1,1-Dimethylethoxy;
$C_1$-$C_6$-Halogenalkyl, insbesondere $C_1$-$C_4$-Halogenalkyl wie Fluormethyl, Difluormethyl, Trifluormethyl, Chlordifluormethyl, Dichlorfluormethyl, Trichlormethyl, 1-Fluorethyl, 2-Fluorethyl, 2,2-Difluorethyl, 2,2,2-Trifluorethyl, 2-Chlor-2,2-difluorethyl, 2,2-Dichlor-2-fluorethyl, 2,2,2-Trichlorethyl und Pentafluorethyl;
$C_1$-$C_6$-Halogenalkoxy, insbesondere $C_1$-$C_4$-Halogenalkoxy wie Difluormethoxy, Trifluormethoxy, Chlordifluormethoxy, Dichlorfluormethoxy, 1-Fluorethoxy, 2-Fluorethoxy, 2,2-Difluorethoxy, 1,1,2,2-Tetrafluorethoxy, 2,2,2-Trifluorethoxy, 2-Chlor-1,1,2-trifluorethoxy und Pentafluorethoxy;
Nitro, Cyano;
Phenyl oder ein- bis dreifach substituiertes Phenyl, wobei als Substituenten $C_1$-$C_4$-Alkyl, Halogen, $C_1$-$C_5$-Halogenalkyl, $C_1$-$C_4$-Alkoxy oder $C_1$-$C_5$-Halogenalkoxy, z.B. wie im einzelnen für die Reste $R^1$ bis $R^3$ aufgeführt, in Frage kommen.

Besonders bevorzugt stehen $R^1$, $R^2$ und $R^3$ für Wasserstoff, Fluor, Chlor, Brom, Jod, Methyl, Ethyl, Isoproypl, Cyclopropyl, Methoxy, Ethoxy, Isopropyloxy, Chlormethyl, Trifluormethyl, Trifluormethoxy und Phenyl, welches ggf. ein- bis dreifach substituiert sein kann mit Halogen bzw. Alkyl- und substituierten Alkylfunktionen wie o.a., z.B. 4-Chlorphenyl, 3,5-Dichlorphenyl, 3-Trifluormethylphenyl, 4-Methoxyphenyl oder 2-Fluor-4-chlorphenyl;

$R^4$

verzweigtes oder unverzweigtes $C_3$-$C_{10}$-Alkinylalkyl, insbesondere $C_3$-$C_6$-Alkinylalkyl, wie 2-Propinyl, 3-Butinyl, 3-Butin-2-yl, 4-Pentinyl, 1-Pentin-3-yl, 2-Hexinyl, wobei 2-Propinyl, 3-Butinyl und 3-Butin-2-yl besonders bevorzugt sind.

$R^5$

$C_1$-$C_5$-Alkyl wie Methyl, Ethyl, n-Propyl, iso-Propyl, n-Butyl, iso-Butyl, sek. Butyl, tert.-Butyl oder Pentyl sowie die unter $R^4$ aufgeführten Reste.

Die Herstellung der Thiophen-2-carbonsäurederivate I kann in an sich bekannter Weise erfolgen, indem man

a) entsprechend substituierte Thiophencarbonsäuren im Fall von X = $OR^4$ mit Alkoholen $R^4OH$ bzw. im Fall von X = $-NHOR^5$ mit Hydroxylaminen $H_2N$-$OR^5$ ggf. in Gegenwart saurer Katalysatoren oder wasserentziehender Mittel oder

b) entsprechend substituierte Thiophen-2-carbonsäurehalogenide im Fall von X = $OR^4$ mit Alkoholen $R^4OH$ bzw. im Fall von X = $OR^4$ mit Alkoholen $R^4OH$ bzw. im Fall von X = $NHOR^5$ mit Hydroxylaminen $H_2N$-$OR^5$ ggf. in Gegenwart von säurebindenden Mitteln umsetzt.

Die als Ausgangssubstanz benötigten Thiophen-2-carbonsäuren sind bekannt oder lassen sich nach allgemein bekannten chemischen Verfahren (s. Heterocyclic Compounds, Thiophene and its Derivatives, Vol. 44, Part 1, S. 1 ff, New York 1985 oder US-A 3,536,473) herstellen. Die Thiophencarbonsäurehalogenide, von denen die Chloride besonders bevorzugt sind, können in bekannter Weise z.B. wie in Houben-Weyl, Methoden der organischen Chemie S. 463 ff, aus den entsprechenden Carbonsäuren erhalten werden.

Die Alkinole $R^4OH$ sind literaturbekannt, im Handel erhältlich oder z.B. wie in Houben-Weyl, Methoden der organischen Chemie, Band 6/1a, Teil 2, S. 1078-1090, 1980, beschrieben, herstellbar.

Die Veresterung der Säure kann in an sich bekannter Weise, z.B. wie in Houben-Weyl, Methoden der organischen Chemie, Band VIII, S. 516 ff, Georg Thieme Verlag, Stuttgart 1952, beschrieben, vorgenommen werden. So kann durch Zusatz von Katalysatoren wie Schwefelsäure, Halogenwasserstoff, Sulfonsäure, sauren Ionenaustauschen die Reaktion beschleunigt oder das veresterungsgleichgewicht im gewünschten Sinne verschoben werden, indem man dem Reaktionsgemisch die Reaktionsprodukte entzieht, beispielsweise indem man das Wasser durch azeotrope Destillation entfernt oder ein wasserentziehendes Mittel wie Dicyclohexylcarbodiimid hinzufügt.

Ausgehend von Thiophen-2-carbonsäurehalogeniden wie -fluoriden, -chloriden oder -bromiden kann man die Veresterung in Gegenwart von Säureakzeptoren, wie in Houben-Weyl, loc.cit, S. 543 ff, beschrieben, durchführen. Säureakzeptoren sind übliche basische Mittel, insbesondere aliphatische, aromatische und heterocyclische Amine, z.B. Triethylamin, N,N-Dimethylanilin, N,N-Dimethylbenzylamin, Pyridin, Lutidin, 4-Dimethylaminopyridin. Auch Alkalimetallcarbonate wie Natrium-oder Kaliumcarbonat eignen sich als Säureakzeptoren.

Die Umsetzung kann in einem Lösungs- oder Verdünnungsmittel vorgenommen werden. Hierzu sind teilweise die angeführten Säureakzeptoren selbst oder beispielsweise folgende Lösungs- oder Verdünnungsmittel oder Gemische derselben geeignet:
Aliphatische und aromatische, gegebenenfalls chlorierte Kohlenwasserstoffe, wie Petrolether, Benzol, Toluol, Xylol, Benzin, Dichlormethan, Chloroform, Tetrachlormethan, 1,2-Dichlormethan, Chlorbenzol, Ether wie Diethyl- und Di-n-butylether, Methyl-tert.-butylether, Tetrahydrofuran, Dioxan; Ketone beispielsweise Aceton,

Methylethylketon, Methylisopropylketon; ferner Nitrile wie Acetonitril und Propionitril.

Üblicherweise setzt man die Ausgangsstoffe in stöchiometrischem Verhältnis ein. Ein Überschuß des einen oder anderen kann in Einzelfällen aber durchaus vorteilhaft sein.

Die erfindungsgemäßen Thiophencarbonsäureester können außerdem noch nach praktisch allen bekannten Verfahren der Estersynthese hergestellt werden, beispielsweise durch Umsetzung von entsprechenden Carbonsäuresalzen mit Propargylhalogeniden, durch Umesterungsreaktionen (vgl. Houben-Weyl a.a.O, S. 508 bis 628; C. Ferri, Reaktionen der organischen Synthese, S. 446 ff, Georg-Thieme-Verlag, Stuttgart 1978; S. Patai, The Chemistry of Carboxylic Acids and Esters, S. 505 55, Interscience Publishers, London 1969).

Die Herstellung der Säureamide kann durch Aminolyse von Thiophen-2-carbonsäurenin bekannter Weise z.B., wie in Houben-Weyl, Methoden der organischen Chemie, Band E5, Teil 2, 4. Auflage, S. 1144ff, beschrieben erfolgen.

Bevorzugt ist die Aminolyse von reaktiveren Carbonsäurederivaten wie Säurehalogeniden, z.B. Säurefluoriden, -chloriden oder -bromiden in Gegenwart der o.a. Säureakzeptoren. (s. loc. cit.).

Die dazu verwendeten Amine sind literaturbekannt oder z.B. gemäß DE-A 36 15 473 oder DE-A 36 31 071 zugänglich.

Die Thiophen-2-carbonsäurederivate I bzw. die sie enthaltenden herbiziden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten im allgemeinen zwischen 0,1 und 95 Gew.%, vorzugsweise zwischen 0,5 und 90 Gew.%, Wirkstoff.

Beispiele für Formulierungen sind:

I. Man vermischt 90 Gew.-Teile der Verbindung Nr. 1 mit 10 Gew.-Teilen N-Methyl-pyrrolidon und erhält eine Lösung, die zur Anwendung in Form kleinster Tropfen geeignet ist.

II. 20 Gew.-Teile der Verbindung Nr. 3 werden in einer Mischung gelöst, die aus 80 Gew.-Teilen Xylol, 10 Gew.-Teilen des Anlagerungsproduktes von 8 bis 10 Mol Ethylenoxid an 1 mol Ölsäure-N-monoethanolamid, 5 Gew.-Teilen Calciumsalz der Dodecylbenzolsulfonsäure und 5 Gew.-Teilen des Anlagerungsproduktes von 40 Mol Ethylenoxid an 1 Mol Ricinusöl besteht. Durch Ausgießen und feines Verteilen der Lösung in 100 000 Gew.-Teilen Wasser erhält man eine wäßrige Dispersion, die 0,02 Gew.-% des Wirkstoffs enthält.

III. 20 Gew.-Teile der Verbindung Nr. 5 werden in einer Mischung gelöst,

. . die aus 40 Gew.-Teilen Cyclohexanon, 30 Gew.-Teilen Isobutanol, 20 Gew.-Teilen des Anlagerungsproduktes von 7 mol Ethylenoxid an 1 mol Isooctylphenol und 10 Gew.-Teilen des Anlagerungsproduktes von 40 mol Ethylenoxid an 1 mol Ricinusöl besteht. Durch Eingießen und feines Verteilen der Lösung in 100 000 Gew.-Teilen Wasser erhält man eine wäßrige Dispersion, die 0,02 Gew.-% des Wirkstoffs enthält.

IV. 20 Gew.-Teile des Wirkstoffs Nr. 11 werden in einer Mischung gelöst, die aus 25 Gew.-Teilen Cyclohexanon, 65 Gew.-Teilen einer Mineralölfraktion vom Siedepunkt 210 bis 280 °C und 10 Gew.-Teilen des Anlagerungsproduktes von 40 mol Ethylenoxid an 1 mol Ricinusöl besteht. Durch Eingießen und feines Verteilen der Lösung in 100 000 Gew.-Teilen Wasser erhält man eine wäßrige Dispersion, die 0,02 Gew.-% des Wirkstoffs enthält.

V. 20 Gew.-Teile des Wirkstoffs Nr. 30 werden mit 3 Gew.-Teilen des Natriumsalzes der Diisobutylnaphthalin-α-sulfonsäure, 17 Gew.-Teilen des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20 000 Gew.-Teilen Wasser erhält man eine Spritzbrühe, die 0,1 Gew.-% des Wirkstoffs enthält.

VI. 3 Gew.-Teile des Wirkstoffs Nr. 28 werden mit 97 Gew.-Teilen feinteiligem Kaolin vermischt. Man erhält auf diese Weise ein Stäubemittel, das 3 Gew.-.% des Wirkstoffs enthält.

VII. 30 Gew.-Teile des Wirkstoffs Nr. 4 werden mit einer Mischung aus 92 Gew.-Teilen pulverförmigem Kieselsäuregel und 8 Gew.-Teilen Paraffinöl, das auf die Oberfläche diese Kieselsäuregels gesprüht wurde, innig vermischt. Man erhält auf diese Weise eine Aufbereitung des Wirkstoffs mit guter Haftfähigkeit.

VIII.20 Gew.-Teile des Wirkstoffs Nr. 30 werden mit 2 Gew.-Teilen Kalziumsalz der Dodecylbenzolsulfonsäure, 8 Gew.-Teilen Fettalkohol-polyglykolether, 2 Gew.-Teilen Natriumsalz eines Phenol-Harnstoff-Formaldehyd-Kondensates und 68 Gew.-Teilen eines paraffinischen Mineralöls innig vermischt. Man erhält eine stabile ölige Dispersion.

IX. 40 Gew.-Teile des Wirkstoffs Nr. 35 werden in 60 Gew.-Teilen einer Mischung, die aus 93 Gew.-% Xylol und 7 Gew.-% des Anlagerungsproduktes von 8 mol Ethylenoxid an 1 mol Nonylphenol besteht, gelöst. Man erhält eine Lösung, die 40 Gew.-% des Wirkstoffs enthält.

Die Applikation der Mittel kann im Vorauflauf- oder im Nachauflaufverfahren erfolgen. Sind die Wirkstoffe für gewisse Kulturpflanzen weniger verträglich, so können auch Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Jahreszeit, Zielpflanzen und Wachstumsstadium 0,001 bis 3 kg/ha, vorzugsweise 0,01 bis 1 kg/ha.

In Anbetracht des erfaßbaren Wirkungsspektrums zur Unkrautbekämpfung, der Verträglichkeit für Kulturpflanzen oder der erwünschten Beeinflussung des Wachstums derselben sowie angesichts der Vielfalt der Applikationsmethoden können die erfindungsgemäßen Verbindungen in einer großen Zahl von Kulturpflanzen eingesetzt werden. In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |

| Botanischer Name | Deutscher Name |
| --- | --- |
| Brassica rapa var. silvestris | Rüben |
| Camellia sinensis | Teestrauch |
| Carthamus tinctorius | Saflor - Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lactuca sativa | Kopfsalat |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |

| Botanischer Name | Deutscher Name |
|---|---|
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Triticum durum | Hartweizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die Thiophen-2-carbonsäurederivate der Formel I sowohl untereinander als auch mit Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, (Hetero-)aryloxyphenoxypropionsäurederivate (Salze, Ester, Amide), Chinolincarbonsäuren und andere in Betracht.

Außerdem kann es von Nutzen sein, die Thiophen-2-carbonsäurederivate der Formel I bzw. sie enthaltende herbizide Mittel allein oder in Kombination mit anderen Herbiziden oder auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Die Herstellung der erfindungsgemäßen Wirkstoffe wird durch folgende Beispiele erläutert.

Beispiel 1

4,5-Dibrom-thiophen-2-carbonsäure-N-ethoxiamid

6,8 g 4,5-Dibromthiophen-2-carbonsäurechlorid werden portionsweise bei Raumtemperatur zu 6,8 g 0-Ethylhydroxylamin-hydrochlorid in 10 ml Pyridin und 80 ml Tetrahydrofuran eingerührt. Nach 30 Minuten versetzt man mit verdünnter Salzsäure, filtriert das Produkt ab und wäscht mit Wasser nach. Ausbeute: 5,8 g; Fp. 117-119 °C.

Beispiel 2

4-Brom-5-chlor-2-thiophencarbonsäure-(3-butinyl)ester

a) 4-Brom-5-chlor-thiophen-2-carbonsäure-(2'-hydroxyphenyl)ester
50 g 4-Brom-5-chlorthiophen werden in 100 ml Methylenchlorid vorgelegt und bei Raumtemperatur mit 55 g 2,2-Dichlor-1,3-benzodioxol versetzt. Anschließend werden unter Eiskühlung portionsweise 67,5 g Aluminiumtrichlorid eingerührt. Danach wird noch 5 min bei Raumtemperatur nachgerührt und noch ca. 5 Minuten unter Rückfluß erhitzt. Zuletzt wird in Eiswasser eingegossen , durchgerührt und abgesaugt. Nach üblicher Aufarbeitung erhält man 84 g kristallines Rohprodukt vom Schmelzpunkt 145-147 °C das weiter eingesetzt wird.
b) 4-Brom-5-chlor-thiophen-2-carbonsäure
80 g der oben beschriebenen Verbindung werden in einer 20 %igen Kalilaugenlösung (800 ml) eine halbe Stunde unter Rückfluß erhitzt. Nach dem Abkühlen wird durch Zugabe von konzentrierter Salzsäure ausgefällt und abgesaugt. Der Rückstand wird in der Wärme in Wasser durch Zugabe von 20 %iger Kalilauge erneut gelöst und der pH-Wert durch Zugabe von festem Ammoniumchlorid auf ca. 10 eingestellt. Der entstandene Festkörper wird abgesaugt und verworfen. Das Filtrat wird anschließend mit konzentrierter Salzsäure angesäuert und das Präzipitat abgesaugt und getrocknet. Ausbeute: 28,7 g, Fp. > 200 °C.
c) 4-Brom-5-chlor-2-thiophencarbonsäurechlorid
Die Synthese erfolgt in üblicher Weise aus der Carbonsäure mittels Umsetzung mit Thionylchlorid in Toluol/n-Pentan mit katalytischen Mengen Dimethylformamid.

Umsetzung zum Wertprodukt

d) 3,0 g des unter c) hergestellten Säurechlorides werden in 30 ml Tetrahydrofuran mit einer Spatelspitze 4-Dimethylaminopyridin und 1,0 g 3-Butin-1-ol versetzt. Anschließend werden 2,0 ml Triethylamin eingetropft und 10 Stunden bei Raumtemperatur nachgerührt. Nach üblicher Aufarbeitung wurden 2,8 g Ester als Öl erhalten.

Entsprechend können durch Wahl der Ausgangsstoffe und entsprechende Anpassung der Verfahrensbedingungen die nachstehend aufgelisteten Verbindungen erhalten werden.

Tabelle 1: Thiophen-2-carbonsäureester

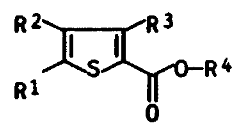

I

| Nr. | $R^1$ | $R^2$ | $R^3$ | $R^4$ | phys. Daten/Fp |
|---|---|---|---|---|---|
| 1 | Cl | Cl | H | $-CH_2-CH_2-C\equiv CH-$ | öl |
| 2 | $C_6H_5$ | Br | H | $-CH_2-C\equiv CH$ | |
| 3 | Br | Br | H | $-CH_2-CH_2-C\equiv CH$ | 50-53°C |
| 4 | Br | Cl | H | $-CH_2-CH_2-C\equiv CH$ | öl |
| 5 | Cl | Br | H | $-CH_2CH_2-C\equiv CH$ | öl |
| 6 | Cl | Br | H | $-CH_2-C\equiv CH$ | |
| 7 | Cl | H | Cl | $-CH_2-C\equiv CH$ | |
| 8 | Br | Br | H | $-CH_2C\equiv CH$ | 60-64°C |
| 9 | $p-ClC_6H_4$ | H | H | $-CH_2C\equiv CH$ | |
| 10 | $m-CF_3-C_6H_4$ | H | H | $-CH_2CH_2C\equiv CH$ | |
| 11 | Br | Br | H | $-CH(CH_3)-C\equiv CH$ | 52-54°C |
| 12 | Br | Br | H | $-CH_2-CH_2-CH_2-C\equiv CH$ | |
| 13 | $CF_3$ | Br | H | $-CH_2-C\equiv CH$ | |

Tabelle 1 - Fortsetzung

| Nr. | $R^1$ | $R^2$ | $R^3$ | $R^4$ | phys. Daten/Fp |
|---|---|---|---|---|---|
| 14 | $C_2H_5O$ | Br | Br | $-CH_2C\equiv CH$ | |
| 15 | $CF_3O$ | Br | H | $-CH_2CH_2C\equiv CH$ | |
| 16 | $n-C_4H_9$ | Br | H | $-CH_2C\equiv CH$ | |
| 17 | $CH_3O$ | Br | Br | $-\underset{\mid \atop CH_3}{CH}-C\equiv CH$ | |
| 18 | $p-F-C_6H_4$ | Br | H | $-\underset{\mid \atop CH_3}{CH}-C\equiv CH$ | |
| 19 | I | H | H | $-CH_2-CH_2-C\equiv CH$ | |
| 20 | F | Br | H | $-\underset{\mid \atop CH_3}{CH}-C\equiv CH$ | |
| 21 | $NO_2$ | H | H | $-CH_2-C\equiv CH$ | |
| 22 | -CN | Br | H | $-CH_2-C\equiv CH$ | |
| 23 | $i-C_3H_7$ | Br | H | $-CH_2-C\equiv CH$ | |
| 24 | H | Br | Br | $-CH_2-CH_2-C\equiv CH$ | |

Tabelle 2: Thiophen-2-carbonsäureamide

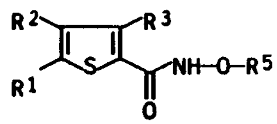

| Nr. | $R^1$ | $R^2$ | $R^3$ | $R^5$ | phys. Daten/Fp |
|---|---|---|---|---|---|
| 25 | Br | Br | H | $C_2H_5$ | 117-119°C |
| 26 | Cl | Cl | H | $-CH_2-C\equiv CH$ | |
| 27 | Cl | Br | H | $C_2H_5$ | 115-118°C |
| 28 | $p-Cl-C_6H_4$ | Br | H | $n-C_3H_7$ | |
| 29 | Br | Cl | H | $C_2H_5$ | 125-126°C |
| 30 | Cl | Cl | Cl | $C_2H_5$ | |
| 31 | Br | Br | H | $-CH-C\equiv CH$<br>$\vert$<br>$CH_3$ | |
| 32 | -CN | Br | H | $C_2H_5$ | |
| 33 | $-NO_2$ | H | H | $-n-C_3H_7$ | |
| 34 | $m-CF_3-C_6H_4$ | H | H | $-n-C_3H_7$ | |
| 35 | $3,5-Cl_2-C_6H_3$ | H | H | $C_2H_5$ | |
| 36 | Cl | Cl | $CH_3$ | $CH_3$ | |
| 37 | Br | Br | H | $CH_2CH_2-C\equiv CH$ | |

Anwendungsbeispiele

Die herbizide Wirkung der Thiophene der Formel I auf das Wachstum der Testpflanzen wird durch folgende Gewächshausversuche gezeigt.

Als Kulturgefäße dienen Plastikblumentöpfe mit 300 $cm^3$ Inhalt und lehmigem Sand mit etwa 3,0 % Humus als Substrat. Die Samen der Testpflanzen werden nach Arten getrennt eingesät.

Zum Zweck der Nachauflaufbehandlung werden entweder direkt gesäte oder in den gleichen Gefäßen aufgewachsene Pflanzen ausgewählt oder sie werden erst als Keimpflanzen getrennt angezogen und einige Tage vor der Behandlung in die Versuchsgefäße verpflanzt.

Je nach Wuchsform werden die Testpflanzen bei einer Wuchshöhe von 3 bis 15 cm dann mit den in Wasser als Verteilungsmittel suspendierten oder emulgierten Wirkstoffen, die durch fein verteilende Düsen gespritzt werden, behandelt. Die Aufwandmenge für die Nachauflaufbehandlung beträgt 1,0 kg/ha a.S. (aktive Substanz).

Die Versuchsgefäße werden im Gewächshaus aufgestellt, wobei für wärmeliebende Arten wärmere Bereiche (20 bis 35 °C) und für solche gemäßigter Klimate 10 bis 20 °C bevorzugt werden. Die Versuchsperiode erstreckt sich über 2 bis 4 Wochen. Während dieser Zeit werden die Pflanzen gepflegt und ihre Reaktion auf die einzelnen Behandlungen wird ausgewertet.

Bewertet wird nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzen sich aus folgenden Arten zusammen:

| Abkürzung | Lateinischer Name | Deutscher Name |
|---|---|---|
| AMARE | Amaranthus retroflexus | Zurückgekrümmter Fuchsschwanz |
| CHEAL | Chenopodium album | Weißer Gänsefuß |
| CHYCO | Chrysantemum corinarium | Kronenwucherblume |
| HELAN | Helianthus annuus | Sonnenblume |
| ORYZA | Oryza sativa | Reis |

Als Vergleichsmittel (A) dient 2,3-Dibromthiophen-5-carbonsäure, die aus dem US-Patent 3 536 473 bekannt ist.

Mit 1,0 kg/ha a.S. (aktive Substanz) im Nachauflaufverfahren eingesetzt, lassen sich mit Beispielen 3 und 8 aus Tabelle I breitblättrige unerwünschte Pflanzen sehr gut bekämpfen bei gleichzeitiger Verträglichkeit für eine Beispielkultur (Tab. II und III).

Tabellen I - III

Bekämpfung unerwünschter breitblättriger Pflanzen und Verträglichkeit für eine Beispielkultur bei Nachauflaufapplikation von 1,0 kg/ha a.S. im Gewächshaus:

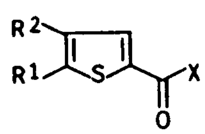

Tabelle I

| Verbindung Nr. | $R_1$ | $R_2$ | X | Testpflanzen und Schädigung % | | |
|---|---|---|---|---|---|---|
| | | | | ORYZA | CHEAL | CHYCO |
| 3 | Br | Br | $O(CH_2)_2C{\equiv}CH$ | 5 | 90 | 100 |
| A | Br | Br | OH | 0 | 60 | 0 |

Tabelle II

| Verbindung Nr. | $R_1$ | $R_2$ | X | Testpflanzen und Schädigung % | | | | |
|---|---|---|---|---|---|---|---|---|
| | | | | ORYZA | CHEAL | HELAN | AMARE | CHYCO |
| 3 | Br | Br | $O(CH_2)_2C \equiv CH$ | 5 | 100 | 100 | 100 | 100 |

Tabelle III

| Verbindung Nr. | $R_1$ | $R_2$ | X | Testpflanzen und Schädigung % | | | |
|---|---|---|---|---|---|---|---|
| | | | | ORYZA | CHEAL | AMARE | CHYCO |
| 8 | Br | Br | $OCH_2C \equiv CH$ | 10 | 100 | 100 | 100 |

**Patentansprüche**
**Patentansprüche für folgende Vertragsstaaten : CH, DE, FR, GB, IT, LI, NL**

**1.** Thiophen-2-carbonsäurederivate der allgemeinen Formel I

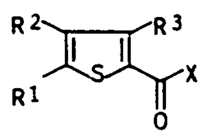

I,

in der die Substituenten folgende Bedeutung haben:

- $R^1$ bis $R^3$ Wasserstoff, Halogen, verzweigtes oder unverzweigtes $C_1$-$C_6$-Alkyl, wobei nicht alle Reste $R^1$ bis $R^3$ für tert.-Butyl stehen, $C_1$-$C_8$-Alkoxy, $C_1$-$C_6$-Halogenalkyl, $C_1$-$C_6$-Halogenalkoxy, Nitro, Cyano, Phenyl oder ein- bis dreifach mit $C_1$-$C_4$-Alkyl, Halogen, $C_1$-$C_5$-Halogenalkyl, $C_1$-$C_4$-Alkoxy oder $C_1$-$C_5$-Halogenalkoxy substituiertes Phenyl;
- X -$OR^4$ oder -NH-$OR^5$, wobei
- $R^4$ verzweigtes oder unverzweigtes $C_3$-$C_{10}$-Alkinylalkyl, das unsubstituiert oder durch Halogen substituiert sein kann;
- $R^5$ verzweigtes oder unverzweigtes $C_1$-$C_5$-Alkyl, das unsubstituiert oder durch Halogen substituiert ist oder verzweigtes oder unverzweigtes $C_3$-$C_{10}$-Alkinylalkyl, das unsubstituiert oder durch Halogen substituiert sein kann;

wobei Thiophen-2-carbonsäurepropargylester der Formel Ia

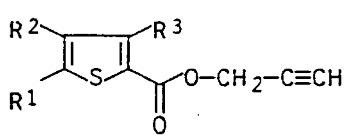

Ia,

ausgenommen sind, worin die Reste $R^1$ bis $R^3$ die folgende Bedeutung haben:

| $R^1$ | $R^2$ | $R^3$ |
|---|---|---|
| H | H | H |
| Cl | H | H |
| $O_2N-$ | H | H |
| Br | H | H |
| Br | Br | H |
| H | H | $H_3C-$ |
| $H_3C-$ | H | H |
| H | Br | H |
| -CN | H | H |
| $H_3C-$ | H | $H_3C-$ |
| $H_3CH_2C-$ | H | H |
| 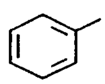 | H | H |
| 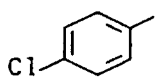 | H | H |
| $H_3CO-$ | H | H |
| H | H | $H_3CO-$ |
| H | H | Cl |
| Cl | H | Cl |
| Cl | Cl | H |
| Cl | Cl | Cl |

**2.** Verfahren zur Herstellung von Thiophen-2-carbonsäurederivaten der allgemeinen Formel I gemäß Anspruch 1, dadurch gekennzeichnet, daß man in an sich bekannter Weise

a) entsprechend substituierte Thiophen-2-carbonsäuren im Fall von X = $OR^4$ mit Alkoholen $R^4OH$ bzw. im Fall von X = $-NHOR^5$ mit Hydroxylaminen $H_2N-OR^5$ ggf. in Gegenwart von wasserentziehenden Mitteln oder

b) entsprechend substituierte Thiophen-2-carbonsäurehalogenide im Fall von X = $OR^4$ mit Alkoholen $R^4OH$ bzw. im Fall von X = $-NHOR^5$ mit Hydroxylaminen $H_2N-OR^5$ ggf. in Gegenwart von säurebindenden Mitteln umsetzt.

**3.** Verfahren nach Anspruch 2, dadurch gekennzeichnet, daß man ein tertiäres Amin als säurebindendes Mittel verwendet.

**4.** Herbizides Mittel, enthaltend neben üblichen inerten Zusatzstoffen ein Thiophen-2-carbonsäurederivat der Formel I.

**5.** Verfahren zur Bekämpfung unerwünschten Pflanzenwuchses, dadurch gekennzeichnet, daß man die unerwünschten Pflanzen und/oder ihren Lebensraum mit einer herbizid wirksamen Menge eines Thiophen-2-carbonsäurederivates der Formel I

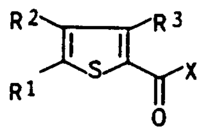

I,

in der die Substituenten folgende Bedeutung haben:

| | |
|---|---|
| $R^1$ bis $R^3$ | Wasserstoff, Halogen, verzweigtes oder unverzweigtes $C_1$-$C_6$-Alkyl, wobei nicht alle Reste $R^1$ bis $R^3$ für tert.-Butyl stehen, $C_1$-$C_8$-Alkoxy, $C_1$-$C_6$-Halogenalkyl, $C_1$-$C_6$-Halogenalkoxy, Nitro, Cyano, Phenyl oder ein- bis dreifach mit $C_1$-$C_4$-Alkyl, Halogen, $C_1$-$C_5$-Halogenalkyl, $C_1$-$C_4$-Alkoxy oder $C_1$-$C_5$-Halogenalkoxy substituiertes Phenyl; |
| X | -$OR^4$ oder -NH-$OR^5$, |
| $R^4$ | verzweigtes oder unverzweigtes $C_3$-$C_{10}$-Alkinylalkyl, das unsubstituiert oder durch Halogen substituiert sein kann; |
| $R^5$ | verzweigtes oder unverzweigtes $C_1$-$C_5$-Alkyl, das unsubstituiert substituiert ist oder verzweigtes oder unverzweigtes $C_3$-$C_{10}$-Alkinylalkyl, das unsubstituiert oder durch Halogen substituiert sein kann, |

behandelt.

**6.** Verwendung eines Thiophen-2-carbonsäurederivates der Formel I gemäß Anspruch 5 zur Bekämpfung unerwünschten Pflanzenwuchses.

**Patentansprüche für folgenden Vertragsstaat : ES**

**1.** Verfahren zur Herstellung von Thiophen-2-carbonsäurederivaten der allgemeinen Formel I

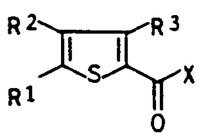

I,

in der die Substituenten folgende Bedeutung haben:

| | |
|---|---|
| $R^1$ bis $R^3$ | Wasserstoff, Halogen, verzweigtes oder unverzweigtes $C_1$-$C_6$-Alkyl, wobei nicht alle Reste $R^1$ bis $R^3$ für tert.-Butyl stehen, $C_1$-$C_8$-Alkoxy, $C_1$-$C_6$-Halogenalkyl, $C_1$-$C_6$-Halogenalkoxy, Nitro, Cyano, Phenyl oder ein- bis dreifach mit $C_1$-$C_4$-Alkyl, Halogen, $C_1$-$C_5$-Halogenalkyl, $C_1$-$C_4$-Alkoxy oder $C_1$-$C_5$-Halogenalkoxy substituiertes Phenyl; |
| X | -$OR^4$ oder -NH-$OR^5$, wobei |
| $R^4$ | verzweigtes oder unverzweigtes $C_3$-$C_{10}$-Alkinylalkyl, das unsubstituiert oder durch Halogen substituiert sein kann; |
| $R^5$ | verzweigtes oder unverzweigtes $C_1$-$C_5$-Alkyl, das unsubstituiert oder durch Halogen substituiert ist oder verzweigtes oder unverzweigtes $C_3$-$C_{10}$-Alkinylalkyl, das unsubstituiert oder durch Halogen substituiert sein kann; |

wobei Thiophen-2-carbonsäurepropargylester der Formel Ia

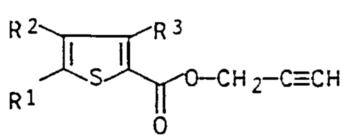

Ia,

ausgenommen sind, worin die Reste $R^1$ bis $R^3$ die folgende Bedeutung haben:

| $R^1$ | $R^2$ | $R^3$ |
|---|---|---|
| H | H | H |
| Cl | H | H |
| $O_2N-$ | H | H |
| Br | H | H |
| Br | Br | H |
| H | H | $H_3C-$ |
| $H_3C-$ | H | H |
| H | Br | H |
| -CN | H | H |
| $H_3C-$ | H | $H_3C-$ |
| $H_3CH_2C-$ | H | H |
| 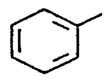 | H | H |
| 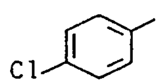 | H | H |
| $H_3CO-$ | H | H |
| H | H | $H_3CO-$ |
| H | H | Cl |
| Cl | H | Cl |
| Cl | Cl | H |
| Cl | Cl | Cl |

dadurch gekennzeichnet, daß man in an sich bekannter Weise

a) entsprechend substituierte Thiophen-2-carbonsäuren im Fall von X = $OR^4$ mit Alkoholen $R^4OH$ bzw. im Fall von X = $-NHOR^5$ mit Hydroxylaminen $H_2N-OR^5$ ggf. in Gegenwart von wasserentziehenden Mitteln oder

b) entsprechend substituierte Thiophen-2-carbonsäurehalogenide im Fall von X = $OR^4$ mit Alkoholen $R^4OH$ bzw. im Fall von X = $-NHOR^5$ mit Hydroxylaminen $H_2N-OR^5$ ggf. in Gegenwart von säurebindenden Mitteln umsetzt.

**2.** Verfahren nach Anspruch 1, dadurch gekennzeichnet, daß man ein tertiäres Amin als säurebindendes Mittel verwendet.

**3.** Herbizides Mittel, enthaltend neben üblichen inerten Zusatzstoffen ein Thiophen-2-carbonsäurederivat der Formel I.

**4.** Verfahren zur Bekämpfung unerwünschten Pflanzenwuchses, dadurch gekennzeichnet, daß man die unerwünschten Pflanzen und/oder ihren Lebensraum mit einer herbizid wirksamen Menge eines Thiophen-2-carbonsäurederivates der Formel I

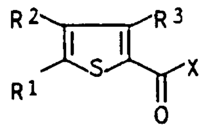

I,

in der die Substituenten folgende Bedeutung haben:

$R^1$ bis $R^3$ Wasserstoff, Halogen, verzweigtes oder unverzweigtes $C_1$-$C_6$-Alkyl, wobei nicht alle Reste $R^1$ bis $R^3$ für tert.-Butyl stehen, $C_1$-$C_8$-Alkoxy, $C_1$-$C_6$-Halogenalkyl, $C_1$-$C_6$-Halogenalkoxy, Nitro, Cyano, Phenyl oder ein- bis dreifach mit $C_1$-$C_4$-Alkyl, Halogen, $C_1$-$C_5$-Halogenalkyl, $C_1$-$C_4$-Alkoxy oder $C_1$-$C_5$-Halogenalkoxy substituiertes Phenyl;

X -$OR^4$ oder -NH-$OR^5$,

$R^4$ verzweigtes oder unverzweigtes $C_3$-$C_{10}$-Alkinylalkyl, das unsubstituiert oder durch Halogen substituiert sein kann;

$R^5$ verzweigtes oder unverzweigtes $C_1$-$C_5$-Alkyl, das unsubstituiert substituiert ist oder verzweigtes oder unverzweigtes $C_3$-$C_{10}$-Alkinylalkyl, das unsubstituiert oder durch Halogen substituiert sein kann,

behandelt.

**5.** Verwendung eines Thiophen-2-carbonsäurederivates der Formel I gemäß Anspruch 5 zur Bekämpfung unerwünschten Pflanzenwuchses.

## Claims

## Claims for the following Contracting States : CH, DE, FR, GB, IT, LI, NL

**1.** A thiophene-2-carboxylic acid derivative of the general formula I

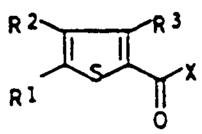

I,

where

$R^1$ to $R^3$ are each hydrogen, halogen, branched or straight-chain $C_1$-$C_6$-alkyl, not all the radicals $R^1$ to $R^3$ being tert-butyl, $C_1$-$C_8$-alkoxy, $C_1$-$C_6$-haloalkyl, $C_1$-$C_6$-haloalkoxy, nitro, cyano, unsubstituted phenyl or phenyl which is mono- to trisubstituted by $C_1$-$C_4$-alkyl, halogen, $C_1$-$C_5$-haloalkyl, $C_1$-$C_4$-alkoxy or $C_1$-$C_5$-haloalkoxy, and

x is -$OR^4$ or -NH-$OR^5$,

$R^4$ being branched or straight-chain $C_3$-$C_{10}$-alkynylalkyl which may be unsubstituted or substituted by halogen, and

$R^5$ being branched or straight-chain $C_1$-$C_5$-alkyl which is unsubstituted or substituted by halogen, or branched or straight-chain $C_3$-$C_{10}$-alkynylalkyl which may be unsubstituted or substituted by halogen,

with the exception of propargyl thiophene-2-carboxylates of the formula Ia

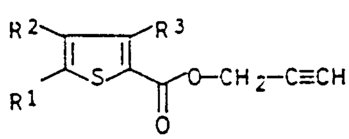

Ia,

where $R^1$ to $R^3$ have the following meanings:

| $R^1$ | $R^2$ | $R^3$ |
|---|---|---|
| H | H | H |
| Cl | H | H |
| $O_2N-$ | H | H |
| Br | H | H |
| Br | Br | H |
| H | H | $H_3C-$ |
| $H_3C-$ | H | H |
| H | Br | H |
| -CN | H | H |
| $H_3C-$ | H | $H_3C-$ |
| $H_3CH_2C-$ | H | H |
| 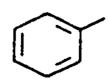 | H | H |
| 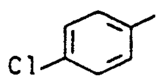 | H | H |
| $H_3CO-$ | H | H |
| H | H | $H_3CO-$ |
| H | H | Cl |
| Cl | H | Cl |
| Cl | Cl | H |
| Cl | Cl | Cl |

2. A process for the preparation of a thiophene-2-carboxylic acid derivative of the general formula I as claimed in claim 1, which comprises, in a known manner,
   a) reacting an appropriately substituted thiophene-2-carboxylic acid with an alcohol $R^4OH$ when X is $OR^4$ or with a hydroxylamine $H_2N-OR^5$ when X is $-NHOR^5$, in the presence or absence of a dehydrating agent, or
   b) reacting an appropriately substituted thiophene-2-carbonyl halide with an alcohol $R^4OH$ when X is $OR^4$ or with a hydroxylamine $H_2N-OR^5$ when X is $-NHOR^5$, in the presence or absence of an acid acceptor.

3. A process as claimed in claim 2, wherein a tertiary amine is used as the acid acceptor.

4. A herbicide containing a thiophene-2-carboxylic acid derivative of the formula I in addition to conventional inert additives.

5. A method for controlling undesirable plant growth, which comprises treating the undesirable plants and/or their habitat with a herbicidal amount of a thiophene-2-carboxylic acid derivative of the formula I

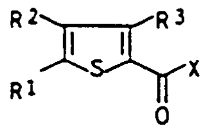

I,

where

$R^1$ to $R^3$ are each hydrogen, halogen, branched or straight-chain $C_1$-$C_6$-alkyl, not all the radicals $R^1$ to $R^3$ being tert-butyl, $C_1$-$C_8$-alkoxy, $C_1$-$C_6$-haloalkyl, $C_1$-$C_6$-haloalkoxy, nitro, cyano, unsubstituted phenyl or phenyl which is mono- to trisubstituted by $C_1$-$C_4$-alkyl, halogen, $C_1$-$C_5$-haloalkyl, $C_1$-$C_4$-alkoxy or $C_1$-$C_5$-haloalkoxy, and

x is $-OR^4$ or $-NH-OR^5$,

$R^4$ being branched or straight-chain $C_3$-$C_{10}$-alkynylalkyl which may be unsubstituted or substituted by halogen, and

$R^5$ being branched or straight-chain $C_1$-$C_5$-alkyl which is unsubstituted or substituted by halogen, or branched or straight-chain $C_3$-$C_{10}$-alkynylalkyl which may be unsubstituted or substituted by halogen.

**6.** The use of a thiophene-2-carboxylic acid derivative of the formula I as claimed in claim 5 for controlling undesirable plant growth.

**Claims for the following Contracting State : ES**

**1.** A process for the preparation of a thiophene-2-carboxylic acid derivative of the general formula I

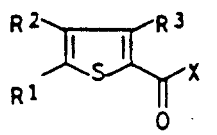

I,

where

$R^1$ to $R^3$ are each hydrogen, halogen, branched or straight-chain $C_1$-$C_6$-alkyl, not all the radicals $R^1$ to $R^3$ being tert-butyl, $C_1$-$C_8$-alkoxy, $C_1$-$C_6$-haloalkyl, $C_1$-$C_6$-haloalkoxy, nitro, cyano, unsubstituted phenyl or phenyl which is mono- to trisubstituted by $C_1$-$C_4$-alkyl, halogen, $C_1$-$C_5$-haloalkyl, $C_1$-$C_4$-alkoxy or $C_1$-$C_5$-haloalkoxy, and

x is $-OR^4$ or $-NH-OR^5$,

$R^4$ being branched or straight-chain $C_3$-$C_{10}$-alkynylalkyl which may be unsubstituted or substituted by halogen, and

$R^5$ being branched or straight-chain $C_1$-$C_5$-alkyl which is unsubstituted or substituted by halogen, or branched or straight-chain $C_3$-$C_{10}$-alkynylalkyl which may be unsubstituted or substituted by halogen,

with the exception of propargyl thiophene-2-carboxylates of the formula Ia

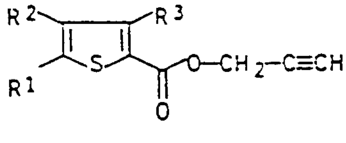

Ia,

where $R^1$ to $R^3$ have the following meanings:

| $R^1$ | $R^2$ | $R^3$ |
|---|---|---|
| H | H | H |
| Cl | H | H |
| $O_2N-$ | H | H |
| Br | H | H |
| Br | Br | H |
| H | H | $H_3C-$ |
| $H_3C-$ | H | H |
| H | Br | H |
| $-CN$ | H | H |
| $H_3C-$ | H | $H_3C-$ |
| $H_3CH_2C-$ | H | H |
| 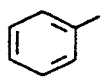 | H | H |
| 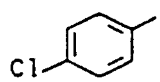 | H | H |
| $H_3CO-$ | H | H |
| H | H | $H_3CO-$ |
| H | H | Cl |
| Cl | H | Cl |
| Cl | Cl | H |
| Cl | Cl | Cl |

which comprises, in a known manner,

a) reacting an appropriately substituted thiophene-2-carboxylic acid with an alcohol $R^4OH$ when X is $OR^4$ or with a hydroxylamine $H_2N-OR^5$ when X is $-NHOR^5$, in the presence or absence of a dehydrating agent, or

b) reacting an appropriately substituted thiophene-2-carbonyl halide with an alcohol $R^4OH$ when X is $OR^4$ or with a hydroxylamine $H_2N-OR^5$ when X is $-NHOR^5$, in the presence or absence of an acid acceptor.

**2.** A process as claimed in claim 1, wherein a tertiary amine is used as the acid acceptor.

**3.** A herbicide containing a thiophene-2-carboxylic acid derivative of the formula I in addition to conventional inert additives.

**4.** A method for controlling undesirable plant growth, which comprises treating the undesirable plants and/or their habitat with a herbicidal amount of a thiophene-2-carboxylic acid derivative of the formula I

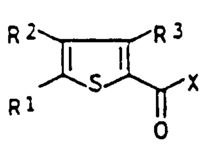

I,

where

$R^1$ to $R^3$ are each hydrogen, halogen, branched or straight-chain $C_1$-$C_6$-alkyl, not all the radicals $R^1$ to $R^3$ being tert-butyl, $C_1$-$C_8$-alkoxy, $C_1$-$C_6$-haloalkyl, $C_1$-$C_6$-haloalkoxy, nitro, cyano, unsubstituted phenyl or phenyl which is mono- to trisubstituted by $C_1$-$C_4$-alkyl, halogen, $C_1$-$C_5$-haloalkyl, $C_1$-$C_4$-alkoxy or $C_1$-$C_5$-haloalkoxy, and

x is $-OR^4$ or $-NH-OR^5$,

$R^4$ being branched or straight-chain $C_3$-$C_{10}$-alkynylalkyl which may be unsubstituted or substituted by halogen, and

$R^5$ being branched or straight-chain $C_1$-$C_5$-alkyl which is unsubstituted or substituted by halogen, or branched or straight-chain $C_3$-$C_{10}$-alkynylalkyl which may be unsubstituted or substituted by halogen.

**5.** The use of a thiophene-2-carboxylic acid derivative of the formula I as claimed in claim 4 for controlling undesirable plant growth.

## Revendications

**Revendications pour les Etats contractants suivants : CH, DE, FR, GB, IT, LI, NL**

**1.** Dérivés d'acide thiophène-2-carboxylique de la formule générale I

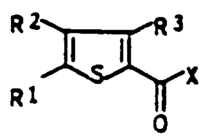

I,

dans laquelle les substituants ont la signification suivante :

$R^1$ à $R^3$, hydrogène, halogène, alkyle en C1-C6 ramifié ou non, tous les restes $R^1$ à $R^3$ n'étant pas mis pour tert-butyle, alcoxy en C1-C8, halogénoalkyle en C1-C6, halogénoalcoxy en C1-C6, nitro, cyano, phényle ou phényle substitué une à trois fois par alkyle en C1-C4, halogène, halogénoalkyle en C1-C5, alcoxy en C1-C4 ou halogénoalcoxy en C1-C5

X $-OR^4$ ou $-NH-OR^5$,

$R^4$, alcynylalkyle en C3-C10 ramifié ou non, qui peut être non substitué ou substitué par halogène,

$R^5$, alkyle en C1-C5 ramifié ou non, qui peut être non substitué ou substitué par halogène, ou alcynylalkyle en C3-C10 ramifié ou non, qui peut être non substitué ou substitué par halogène ;

les esters propargyliques d'acide thiophène-2-carboxylique de formule Ia

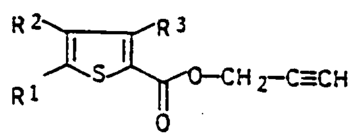

Ia,

étant exclus, dans lesquels les restes R1 à R3 ont les significations suivantes :

| R1 | R2 | R3 |
|---|---|---|
| H | H | H |
| Cl | H | H |
| $O_2N-$ | H | H |
| Br | H | H |
| Br | Br | H |
| H | H | $H_3C-$ |
| $H_3C-$ | H | H |
| H | Br | H |
| -CN | H | H |
| $H_3C-$ | H | $H_3C-$ |
| $H_3CH_2C-$ | H | H |
| 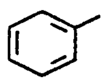 | H | H |
| 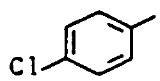 | H | H |
| $H_3CO-$ | H | H |
| H | H | $H_3CO-$ |
| H | H | Cl |
| Cl | H | Cl |
| Cl | Cl | H |
| Cl | Cl | Cl |

2. Procédé de préparation de dérivés d'acide thiophène-2-carboxylique de la formule générale I selon la revendication 1, caractérisé par le fait que l'on fait réagir, de manière connue en soi :
   a) des acides thiophène-2-carboxyliques substitués en correspondance, avec des alcools $R^4OH$ lorsque X = $OR^4$ ou avec des hydroxylamines $H_2N\text{-}OR^5$ lorsque X = $-NHOR^5$ éventuellement en présence d'agents déshydratants, ou
   b) des halogénures d'acides thiophène-2-carboxyliques substitués en correspondance, avec des alcools $R^4OH$ lorsque X = $OR^4$ ou avec des hydroxylamines $H_2N\text{-}OR^5$ lorsque X = $-NHOR^5$ éventuellement en présence d'agents déshydratants liant les acides.

3. Procédé selon la revendication 2, caractérisé par le fait que l'on utilise une amine tertiaire comme agent liant les acides.

4. Herbicide contenant à côté d'additifs inertes usuels, un dérivé d'acide thiophène-2-carboxylique de la formule générale I.

5. Procédé pour lutter contre la croissance de plantes indésirables, caractérisé par le fait que l'on traite les plantes indésirables et/ou leur biotope avec une quantité efficace comme herbicide d'un dérivé d'acide thiophène-2-carboxylique de la formule générale I

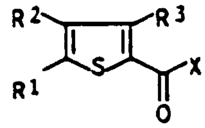

I,

dans laquelle les substituants ont la signification suivante :

$R^1$ à $R^3$, hydrogène, halogène, alkyle en C1-C6 ramifié ou non, tous les restes $R^1$ à $R^3$ n'étant pas mis pour tert-butyle, alcoxy en C1-C8, halogénoalkyle en C1-C6, halogénoalcoxy en C1-C6, nitro, cyano, phényle ou phényle substitué une à trois fois par alkyle en C1-C4, halogène, halogénoalkyle en C1-C5, alcoxy en C1-C4 ou halogénoalcoxy en C1-C5

X -$OR^4$ ou -NH-$OR^5$,

$R^4$, alcynylalkyle en C3-C10 ramifié ou non, qui peut être non substitué ou substitué par halogène,

$R^5$, alkyle en C1-C5 ramifié ou non, qui peut être non substitué ou substitué par halogène, ou alcynylalkyle en C3-C10 ramifié ou non, qui peut être non substitué ou substitué par halogène.

**6.** Utilisation d'un dérivé d'acide thiophène-2-carboxylique de la formule générale I selon la revendication 5 pour lutter contre la croissance des plantes indésirables.

**Revendications pour l'Etat contractant suivant : ES**

**1.** Procédé de préparation de dérivés d'acide thiophène-2-carboxylique de la formule générale I

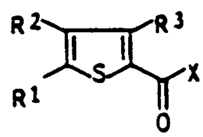

I,

dans laquelle les substituants ont la signification suivante :

$R^1$ à $R^3$, hydrogène, halogène, alkyle en C1-C6 ramifié ou non, tous les restes $R^1$ à $R^3$ n'étant pas mis pour tert-butyle, alcoxy en C1-C8, halogénoalkyle en C1-C6, halogénoalcoxy en C1-C6, nitro, cyano, phényle ou phényle substitué une à trois fois par alkyle en C1-C4, halogène, halogénoalkyle en C1-C5, alcoxy en C1-C4 ou halogénoalcoxy en C1-C5

X -$OR^4$ ou -NH-$OR^5$,

$R^4$, alcynylalkyle en C3-C10 ramifié ou non, qui peut être non substitué ou substitué par halogène,

$R^5$, alkyle en C1-C5 ramifié ou non, qui peut être non substitué ou substitué par halogène, ou alcynylalkyle en C3-C10 ramifié ou non, qui peut être non substitué ou substitué par halogène ;

les esters propargyliques d'acide thiophène-2-carboxylique de formule Ia

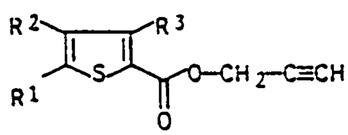

Ia,

étant exclus, dans lesquels les restes R1 à R3 ont les significations suivantes :

| $R^1$ | $R^2$ | $R^3$ |
|---|---|---|
| H | H | H |
| Cl | H | H |
| $O_2N-$ | H | H |
| Br | H | H |
| Br | Br | H |
| H | H | $H_3C-$ |
| $H_3C-$ | H | H |
| H | Br | H |
| -CN | H | H |
| $H_3C-$ | H | $H_3C-$ |
| $H_3CH_2C-$ | H | H |
| 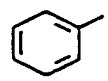 | H | H |
| 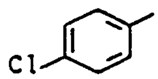 | H | H |
| $H_3CO-$ | H | H |
| H | H | $H_3CO-$ |
| H | H | Cl |
| Cl | H | Cl |
| Cl | Cl | H |
| Cl | Cl | Cl |

caractérisé par le fait que l'on fait réagir, de manière connue en soi :

a) des acides thiophène-2-carboxyliques substitués en correspondance, avec des alcools $R^4OH$ lorsque X = $OR^4$ ou avec des hydroxylamines $H_2N-OR^5$ lorsque X = $-NHOR^5$ éventuellement en présence d'agents déshydratants, ou

b) des halogénures d'acides thiophène-2-carboxyliques substitués en correspondance, avec des alcools $R^4OH$ lorsque X = $OR^4$ ou avec des hydroxylamines $H_2N-OR^5$ lorsque X = $-NHOR^5$ éventuellement en présence d'agents déshydratants liant les acides.

**2.** Procédé selon la revendication 1, caractérisé par le fait que l'on utilise une amine tertiaire comme agent liant les acides.

**3.** Herbicide contenant à côté d'additifs inertes usuels, un dérivé d'acide thiophène-2-carboxylique de la formule générale I.

**4.** Procédé pour lutter contre la croissance de plantes indésirables, caractérisé par le fait que l'on traite les plantes indésirables et/ou leur biotope avec une quantité efficace comme herbicide d'un dérivé d'acide thiophène-2-carboxylique de la formule générale I

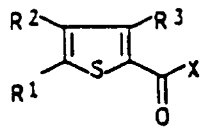

I,

dans laquelle les substituants ont la signification suivante :

| | |
|---|---|
| $R^1$ à $R^3$, | hydrogène, halogène, alkyle en C1-C6 ramifié ou non, tous les restes $R^1$ à $R^3$ n'étant pas mis pour tert-butyle, alcoxy en C1-C8, halogénoalkyle en C1-C6, halogénoalcoxy en C1-C6, nitro, cyano, phényle ou phényle substitué une à trois fois par alkyle en C1-C4, halogène, halogénoalkyle en C1-C5, alcoxy en C1-C4 ou halogénoalcoxy en C1-C5 |
| X | $-OR^4$ ou $-NH-OR^5$, |
| $R^4$, | alcynylalkyle en C3-C10 ramifié ou non, qui peut être non substitué ou substitué par halogène, |
| $R^5$, | alkyle en C1-C5 ramifié ou non, qui peut être non substitué ou substitué par halogène, ou alcynylalkyle en C3-C10 ramifié ou non, qui peut être non substitué ou substitué par halogène. |

**5.** Utilisation d'un dérivé d'acide thiophène-2-carboxylique de la formule générale I selon la revendication 5 pour lutter contre la croissance des plantes indésirables.